(19) Europäisches Patentamt
European Patent Office
Office européen des brevets

(11) Veröffentlichungsnummer: **0 018 324**
**A1**

# (12) EUROPÄISCHE PATENTANMELDUNG

(21) Anmeldenummer: **80810118.2**

(22) Anmeldetag: **03.04.80**

(51) Int. Cl.³: **C 07 C 43/257**, C 07 C 41/16, C 07 C 125/065, C 07 C 121/75, C 07 C 149/00, C 07 C 147/00, C 07 C 69/96, C 07 C 69/712, C 07 D 213/64, A 01 N 31/16, A 01 N 33/04

(30) Priorität: **12.04.79 CH 3531/79**

(43) Veröffentlichungstag der Anmeldung: **29.10.80 Patentblatt 80/22**

(84) Benannte Vertragsstaaten: **AT BE CH DE FR GB IT LI NL**

(71) Anmelder: **CIBA-GEIGY AG, Patentabteilung Postfach, CH-4002 Basel (CH)**

(72) Erfinder: **Rohr, Otto, Dr., Kilbertweg 19, CH-4106 Therwil (CH)**
Erfinder: **Pissiotas, Georg, Dr., Breslauerstrasse 8, D-7850 Lörrach (DE)**
Erfinder: **Böhner, Beat, Dr., Hügelweg 3, CH-4102 Binningen (CH)**
Erfinder: **Szczepanski, Henry, Dr., Waldshuterstrasse 55, CH-4310 Rheinfelden (CH)**

(54) **Herbizid wirksame substituierte Diphenylätherderivate, Verfahren zu ihrer Herstellung sowie herbizide Mittel und deren Verwendung.**

(57) Die Erfindung betrifft neue herbizid wirksame Diphenylätherderivate, diese als Wirkstoffe enthaltende herbizide Mittel, welche sich insbesondere zur Bekämpfung von Ungräsern in Kulturpflanzungen eignen.

Die neuen Wirkstoffe haben die Formel

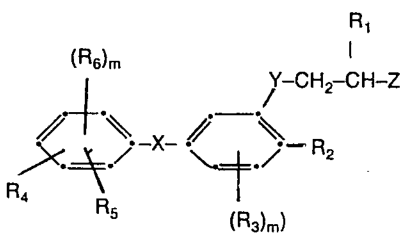

worin $R_1$ niederes Alkyl, $R_2$ Wasserstoff, Halogen oder Cyano, $R_3$ Halogen, niederes Alkyl oder Nitro, $R_4$ und $R_5$ je Wasserstoff, $CF_3$, CN oder $NO_2$, $R_6$ Halogen, m eine Zahl von 0 bis 3, X und Y Schwefel oder vorzugsweise Sauerstoff, und

Z ist Halogen, –OR, $-\overset{}{S}-R$ mit $\|$ $(O)_n$ oder 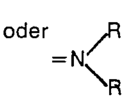

wobei n = Null, 1 oder 2 und R einen gegebenenfalls substituierten aliphatischen oder aromatischen Rest oder Wasserstoff darstellt.

EP 0 018 324 A1

0018324

CIBA-GEIGY AG
Basel (Schweiz)

5-12309/=

# Herbizid wirksame substituierte Diphenylätherderivate, Verfahren zu ihrer Herstellung sowie herbizide Mittel und deren Verwendung

Die vorliegende Erfindung betrifft neue, herbizid wirksame substituierte Diphenylätherderivate, Verfahren zu ihrer Herstellung, ferner herbizide Mittel, die diese neuen Wirkstoffe enthalten, sowie die Verwendung der neuen Derivate und der sie enthaltenden Mittel zur selektiven Bekämpfung von Unkräutern in Kulturpflanzenbeständen.

In der Literatur sind neben zahlreichen, in verschiedener Weise substituierten (Phenoxy)-α-phenoxyalkancarbonsäurederivaten auch substituierte 3- und 4-(Phenoxy)-phenoxy-alkanole, ihre entsprechenden Phenylthio-Analogen, Mercaptane, Ester sowie Amine und weitere Derivate bekannt geworden (DOS 2 611 695, DOS 2 533 172 und USP 4 046 798).

Es wurde nun gefunden, dass gewisse 3-(Phenoxy)-phenoxyalkanole und deren Abkömmlinge diesen vorbekannten Verbindungen in mancher Hinsicht in der herbiziden Wirksamkeit überlegen sind.

Die neuen Wirkstoffe vorliegender Erfindung entsprechen der Formel I

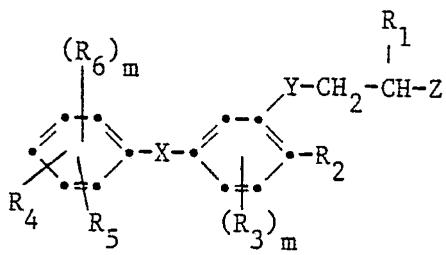

(I)

in welcher die Symbole folgende Bedeutungen haben:

$R_1$ ist $C_1$-$C_4$ Alkyl, $R_2$ ist Wasserstoff, Halogen oder die Cyanogruppe, $R_3$ ist Wasserstoff, Halogen, $C_1$-$C_4$ Alkyl oder die Nitrogruppe, $R_4$ und $R_5$ bedeuten unabhängig voneinander je Wasserstoff, $CF_3$, CN oder $NO_2$, $R_6$ ist Wasserstoff oder Halogen, m ist eine Zahl von 0 bis 3, X und Y sind unabhängig voneinander Sauerstoff oder Schwefel, Z ist Halogen oder eine Gruppe -OH, -SH, $-OR_7$, $-\underset{(O)n}{\overset{}{S}}-R_7-$ oder 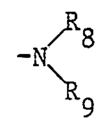 , dabei $R_7$ Alkyl, Cycloalkyl, Alkenyl, Cycloalkenyl, Alkinyl, Phenyl oder Benzyl darstellt, wobei die genannten Reste auch durch Halogen, $CF_3$, $NO_2$, CN, Alkyl, Alkylthio, Alkoxy, -OH, $NH_2$, Alkylamino, Dialkylamino und/oder Alkoxycarbonyl substituiert sein können, $R_7$ ferner eine -CONH-Alkyl, -CO-NH-Aryl, -CO-N(Dialkyl), -CO-Aryl oder -CO-Alkyl-Gruppe darstellen kann, wobei die Alkyl- und Arylreste noch durch Halogen substituiert sein können, $R_7$ schliesslich auch einen Rest

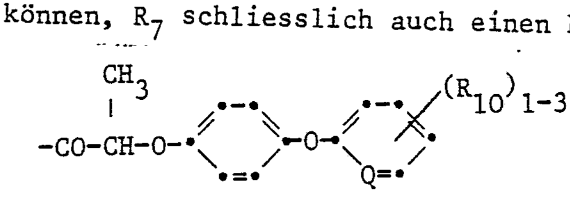

bedeuten kann, in welchem der oder die Substituenten $R_{10}$ unabhängig voneinander Halogen, $NO_2$, CN, $CF_3$ oder Alkyl darstellen und Q durch =CH- oder =N- verkörpert ist, n die Zahl 0, 1 oder 2, $R_8$ und $R_9$ sind unabhängig voneinander je Wasserstoff, $C_1$-$C_4$ Alkyl, das unsubstituiert oder durch Halogen, CN, -OH oder $C_1$-$C_4$ Alkoxy substituiert sein kann, oder $R_8$ und $R_9$ bilden zusammen mit dem benachbarten Stickstoffatom einen 5- bis 6-gliedrigen heterocyclischen Ring, der auch noch ein Sauerstoff-, Schwefel- oder weiteres Stickstoffatom als

zweites Heteroatom enthalten und durch $C_1$-$C_4$ Alkyl oder durch Phenyl substituiert sein kann.

Die erfindungsgemässen Wirkstoffe der Formel I können ausgehend von Phenoxyphenolen bzw. Phenylthio-(thio)phenolen der Formel II

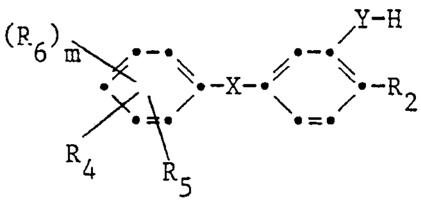

(II)

oder entsprechenden Phenolaten nach vielfachen, dem Fachmann geläufigen Methoden hergestellt werden, z.B. durch Umsetzung mit Verbindungen der Formel

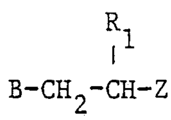

worin B Halogen oder eine Sulfoestergruppe bedeutet, wie z.B. 1-Brompropanol, gegebenenfalls in Gegenwart säurebindender Mittel und anschliessende Veresterung der so erhaltenen Propanole.

Alkohole (Z=OH) stellt man auch her durch Verseifung der entsprechenden Halogenalkylverbindungen (Z=Halogen), welche aus Alkoholen und Halogenierungsmitteln wie Thionylchlorid erhalten wurden;

Ferner erhält man Alkohole durch Reduktion entsprechender Carbonsäureester, z.B. mit $LiAlH_4$ oder $LiBH_4$.

Merkaptane (Z=SH) erhält man beispielsweise aus den entsprechenden Halogenverbindungen (Z=Halogen) durch Umsetzung mit Metallhydrogensulfiden bzw. -xanthaten oder durch Umsetzung mit Thioharnstoff und anschliessender alkalischer Spaltung der entstandenen Thiuroniumsalze.

Aether und Thioäther (Z=$OR_7$ und $SR_7$) werden hergestellt durch Umsetzung der entsprechenden Halogenverbindungen (Z=Halogen) mit Alkoholen oder Merkaptanen oder durch Umsetzung der entsprechenden Alkohole bzw. Merkaptane (Z=OH oder SH) mit Halogeniden.

Ester bzw. Thioester erhält man in an sich bekannter Weise durch Umsetzung von entsprechenden Alkoholen oder Merkaptanen (Z=OH oder SH) mit Säurehalogeniden, Säureanhydriden oder Carbamoylhalogeniden in Gegenwart säurebindender Mittel.

Sulfoxide oder Sulfone werden erhalten durch Oxydation der entsprechenden Thioäther, beispielsweise mit $H_2O_2$ oder Permanganat.

Amine erhält man durch Umsetzung entsprechender Halogenide (Z=Hal) mit Aminen $HNR_8R_9$.

Die Ausgangsstoffe der Formel II sind zum Teil bekannt. Noch nicht beschriebene Ausgangsstoffe der Formel II lassen sich nach bekannten Methoden herstellen, wie sie in DOS 2 643 438, 2 639 796 usw. beschrieben sind. Falls ein Substituent $R_2$ Halogen oder die Cyanogruppe ist, lässt er sich aus der entsprechenden Nitroverbindung durch Reduktion der Nitro- zur Aminogruppe, Diazotierung derselben und Ersatz der Diazogruppe durch Halogen oder -CN in bekannter Weise einführen.

Die genannten Umsetzungen werden vorzugsweise in einem gegenüber den Reaktionskomponenten inerten Lösungs- oder Verdünnungsmittel durchgeführt. Dazu eignen sich solche aus den verschiedensten Stoffklassen, wie aliphatische und aromatische, gegebenenfalls halogenierte Kohlenwasserstoffe, sowie polare inerte organische Lösungsmittel, wie Aether, Ketone, Amide etc. Als Beispiele seien Diäthyläther, Butanon, Methylenchlorid, Methyl-äthylketon, Dimethoxyäthan, Dimethylformamid und Tetrahydrofuran speziell genannt.

Als basische Säureakzeptoren (Säurebindemittel) für die Umsetzung mit Halogenverbindungen können wässerige Alkalimetallhydroxyde, wie KOH und NaOH sowie Karbonate ($K_2CO_3$, $NaHCO_3$), Alkoholate ($NaOCH_3$), aber insbesondere auch organische Basen wie Triäthylamin, Pyridin etc. verwendet werden.

Bevorzugte Verbindungen der Formel I sind jene, in denen X durch Sauerstoff verkörpert ist, auch Y ist vorzugsweise Sauerstoff, $R_1$ ist vorzugsweise die Methylgruppe.

Die erfindungsgemässen Verbindungen der Formel I besitzen ein breites gutes Wirkungsspektrum insbesondere gegen Ungräser (monocotyle Unkräuter), werden von dicotylen Kulturpflanzen und durch verschiedene Getreidearten aber gut toleriert und können deshalb zur selektiven Bekämpfung von Ungräsern in Kulturpflanzenbeständen eingesetzt werden, und zwar sowohl in pre- als auch in postemergenter Applikation.

Die nachfolgenden Beispiele veranschaulichen die Herstellung einiger erfindungsgemässer Wirkstoffe der Formel I. Weitere in entsprechender Weise hergestellte Wirkstoffe sind anschliessend tabellarisch aufgeführt.

Beispiel 1: 81 g (0,25 Mol) 2,4'-Dichlor-3'-hydroxy-4-trifluormethyl diphenyläther werden in eine Suspension von 11 g pulverisiertem Natriumhydroxyd in 300 ml Dimethylsulfoxid eingetragen. Dazu tropft man 28 g 1-Chlor-2-propanol, erhitzt das Reaktionsgemisch auf 100°C (Badtemperatur) und rührt während 2 Tagen. Anschliessend giesst man in Wasser und extrahiert das Produkt mit Essigester/Toluol 1:1. Das so erhaltene Oel wird im Hochvakuum destilliert, wobei man 83,5 g der Verbindung der Formel

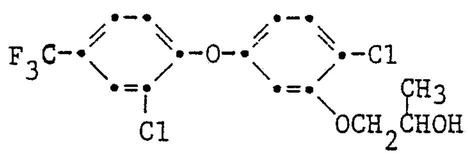

erhält: Kp: 153°-158°C/0,03 Torr.

Auf analoge Weise wurde auch die folgende Verbindung hergestellt

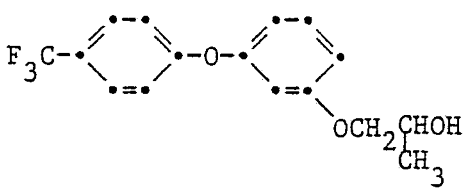

Kp: 133°C/0,015 Torr.

Beispiel 2: 19,6 g 3-(2'-chlor-4'-trifluormethyl-phenoxy)-α-(6-chlor phenoxy)-propanol (Bsp. 1) werden mit 4-(4'-Trifluormethylphenoxy)-α-phenoxy-propionsäure-chlorid in 100 ml Toluol vorgelegt. Dazu tropft man bei 15°-20°C 7,5 g Triäthylamin und rührt 2 Std. bei Raumtemperatur aus. Nach Zugabe von 50 ml Wasser wird die organische Phase abgetrennt, getrocknet und eingedampft. Das erhaltene Oel wird im Kugelrohrofen im Hochvakuum destilliert. Man erhält 25 g der Verbindung:

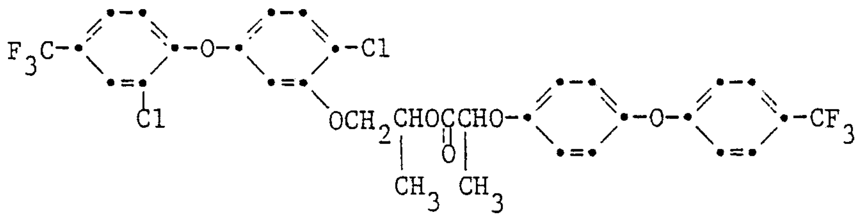

Kp: 230°C/0,001 Torr.

Die in den vorstehenden Beispielen beschriebenen sowie weitere Wirkstoffe der Formel I sind in der nachstehenden Tabelle aufgeführt, und zwar so, dass einerseits die Grundkörper der Formel I und andererseits die dazugehörigen Reste Z gesondert angegeben sind.

A.) Grundkörper

1. 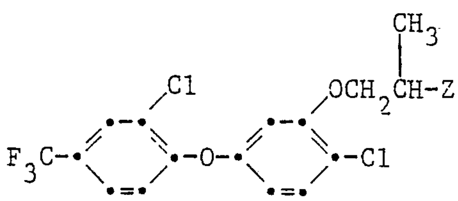

2. 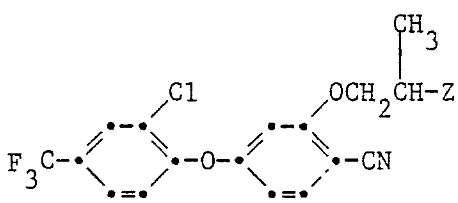

3. 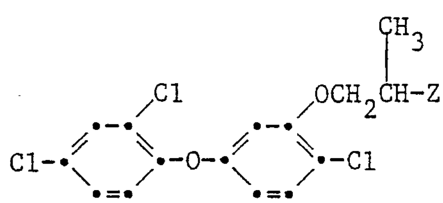

4. 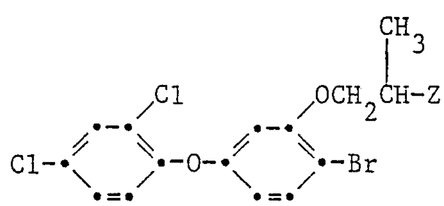

5. 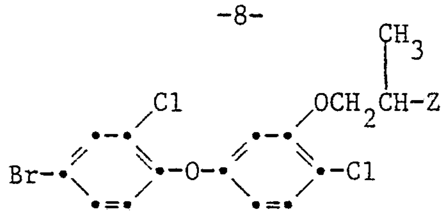

6. 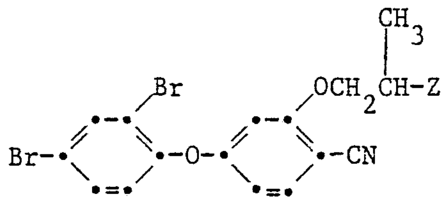

7. 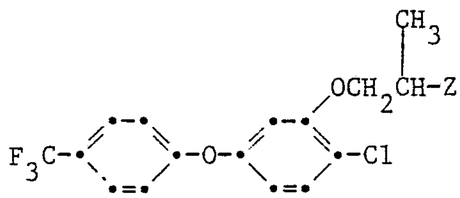

8. 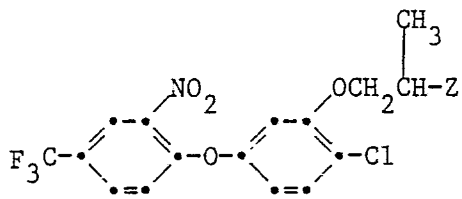

9. 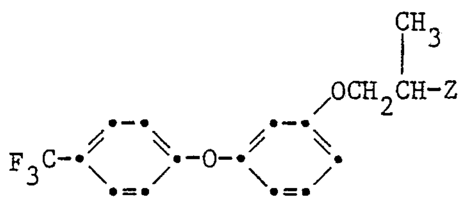

10. 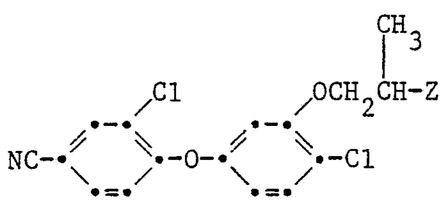

11. 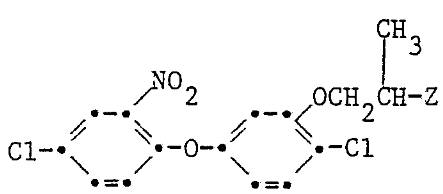

12. 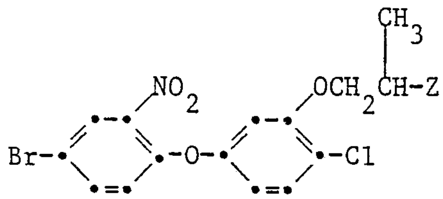

13. 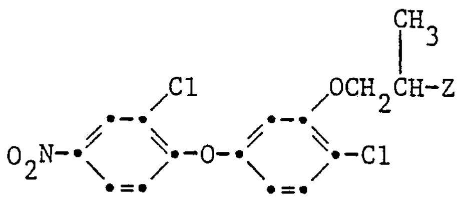

14. 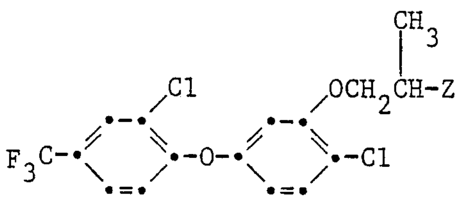

B.) Dazugehörige Reste Z

a) -OH

b) 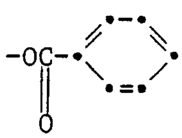

c) 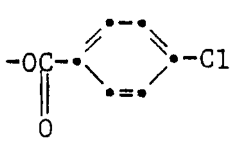

d) 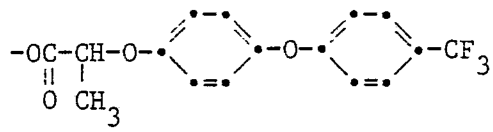

e) 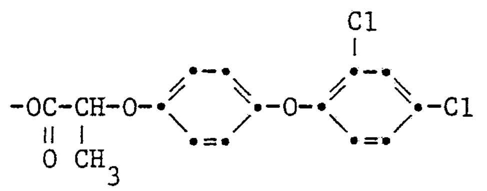

f) 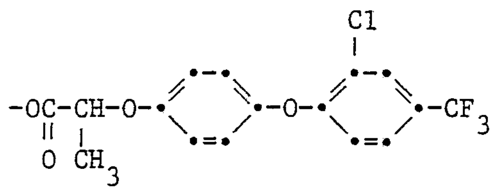

g) 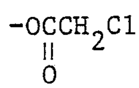

h) 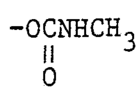

i) 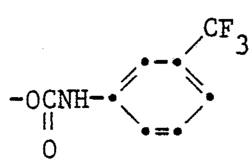

j) 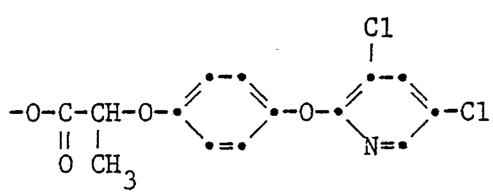

k) 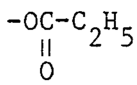

l) $-OSO_2CH_3$

m) 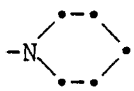

n) 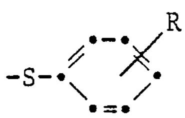 R = Hal, $NO_2$, CN, $CF_3$, Alkyl, Alkoxy.

o) 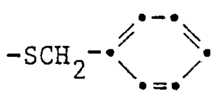

p) $-SCH_2CH=CH_2$

q) $-OCH_2CH=CH_2$

r) $-OCH_2C\equiv CH$

s) $-OCH_3$

t) 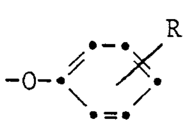 R = Hal, $NO_2$, CN, $CF_3$, Alkyl, Alkoxy.

u) 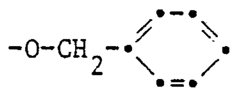

v) 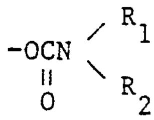 $R_1$, $R_2$ = H, Alkyl, Alkoxy.

w) 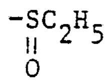

x) 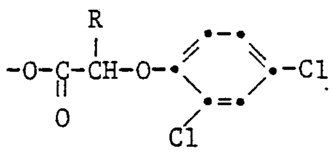 R = H oder $CH_3$

y) 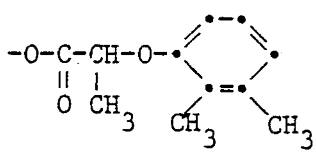

z) 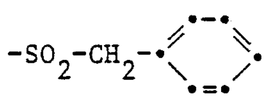

Ausgewählte Einzelwirkstoffe der Formel I zusätzlich zu den in den Beispielen beschriebenen sind nachstehend tabellarisch zusammengestellt. Sie fallen unter die folgende engere Formel:

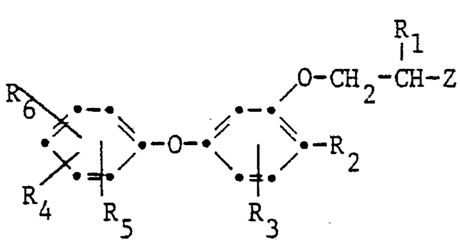

| Verbindung No. | $R_1$ | $R_2$ | $R_3$ | $R_4$ | $R_5$ | $R_6$ | Z | Kp. oder $n_D$ |
|---|---|---|---|---|---|---|---|---|
| 1 | $CH_3$ | Cl | H | 4-$CF_3$ | H | 2-Cl | $-O-CO-OC_2H_5$ | Kp.150°/0,001 Torr |
| 2 | $CH_3$ | Cl | H | 4-$CF_3$ | H | 2-Cl | $-O-CH_2-CH=CH_2$ | Kp.140°/0,001 Torr |
| 3 | $CH_3$ | Cl | H | 4-$CF_3$ | H | 2-Cl | 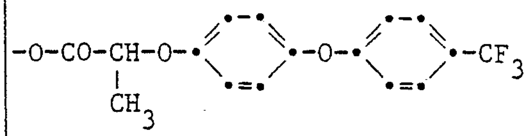 | |
| 4 | $CH_3$ | Cl | H | 4-$CF_3$ | H | 2-Cl | $-O-CO-N(CH_3)-OCH_3$ | Kp.160°/0,001 Torr |
| 5 | $CH_3$ | Cl | H | 4-$CF_3$ | H | 2-Cl | $-O-CO-NH-CH_3$ | $n_D^{27}$ = 1,5275 |
| 6 | $CH_3$ | Cl | H | 4-$CF_3$ | H | 2-Cl | $-O-CO-CH_2Cl$ | $n_D^{27}$ = 1,5282 |
| 7 | $CH_3$ | Cl | H | 4-$CF_3$ | H | 2-Cl | 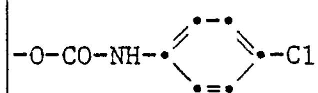 | |
| 8 | $CH_3$ | Cl | H | 4-$CF_3$ | H | 2-Cl | $-O-CO-CHCl_2$ | $n_D^{27}$ = 1,5302 |
| 9 | $CH_3$ | Cl | H | 4-$CF_3$ | H | 2-Cl | 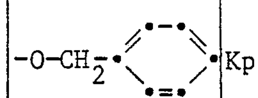 | Kp.170°/0,001 Torr |
| 10 | $CH_3$ | Cl | H | 4-$CF_3$ | H | 2-Cl | 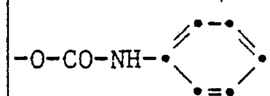 | |
| 11 | $CH_3$ | Cl | H | 4-$CF_3$ | H | 2-Cl | 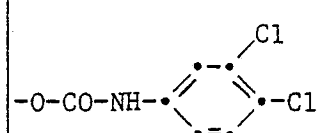 | Kp.180°/0,001 Torr |
| 12 | $CH_3$ | Cl | H | 4-$CF_3$ | H | 2-Cl | $-O-CO-C_2H_5$ | $n_D^{27}$ = 1,5151 |
| 13 | $CH_3$ | Cl | H | 4-$CF_3$ | H | 2-Cl | 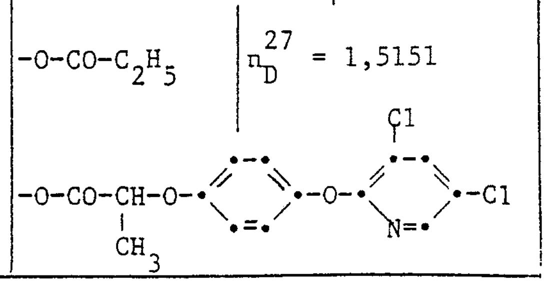 | |

| Verbindung No. | $R_1$ | $R_2$ | $R_3$ | $R_4$ | $R_5$ | $R_6$ | Z | Kp. |
|---|---|---|---|---|---|---|---|---|
| 14 | $CH_3$ | Cl | H | $4-CF_3$ | H | 2-Cl | Cl | Kp.145°/0,04 Torr |
| 15 | $CH_3$ | Cl | H | $4-CF_3$ | H | 2-Cl | $-SCH_3$ | |
| 16 | $CH_3$ | Cl | H | $4-CF_3$ | H | 2-Cl | $-SOCH_3$ | |
| 17 | $CH_3$ | CN | H | $4-CF_3$ | H | 2-Cl | -OH | |
| 18 | $CH_3$ | Cl | 2-Cl | $4-CF_3$ | H | 2-Cl | -OH | |
| 19 | $CH_3$ | Cl | H | $4-CF_3$ | $2-NO_2$ | H | -OH | |
| 20 | $CH_3$ | Cl | H | $4-CF_3$ | 2-CN | H | -OH | |

Die Erfindung betrifft auch herbizide Mittel, welche einen neuen Wirkstoff der Formel I enthalten, sowie Verfahren zur pre- und post-emergenten Unkrautbekämpfung, insbesondere von monocotylen Ungräsern.

Die erfindungsgemässen Mittel können in den üblichen Formulierungen vorliegen.

Die Herstellung erfindungsgemässer Mittel erfolgt in an sich bekannter Weise durch inniges Vermischen und Vermahlen von Wirkstoffen der Formel I mit geeigneten Trägerstoffen und/oder Verteilungsmitteln, gegebenenfalls unter Zusatz von gegenüber den Wirkstoffen inerten Antischaum-, Netz-, Dispersions- oder Lösungsmitteln. Die Wirkstoffe können in den folgenden Aufarbeitungsformen vorliegen und angewendet werden:

feste Aufarbeitungsformen: Stäubemittel, Streumittel, Granulate, Umhüllungsgranulate, Imprägnierungsgranulate und Homogengranulate;

| | |
|---|---|
| in Wasser dispergierbare Wirkstoffkonzentrate: | Spritzpulver, (wettable powder), Pasten, Emulsionen; Emulsionskonzentrate; |
| flüssige Aufarbeitungsformen: | Lösungen. |

Die Wirkstoffkonzentrationen betragen in den erfindungsgemässen Mitteln 1 bis 80 Gew.-% und können vor der Anwendung auch auf niedrige Konzentrationen, wie etwa 0,05 bis 1% verdünnt werden. Die Aufwandmengen betragen in der Regel 0,1 bis 10 kg Wirkstoff pro Hektar, vorzugsweise 0,25 bis 4 kg/ha.

Die Herstellung erfindungsgemässer Mittel wird durch die folgenden Formulierungsbeispiele veranschaulicht. Teile bedeuten darin Gewichtsteile.

Granulat

Zur Herstellung eines 5%igen Granulates werden die folgenden Stoffe verwendet:

| | |
|---|---|
| 5 | Teile Wirkstoff |
| 0,25 | Teile Epichlorhydrin |
| 0,25 | Teile Cetylpoläthylenglykoläther mit 8 Mol Aethylenoxid, |
| 3,50 | Teile Polyäthylenglykol, |
| 91 | Teile Kaolin (Korngrösse 0,3 bis 0,8 mm) |

Die Aktivsubstanz wird mit Epichlorhydrin vermischt und in 6 Teilen Aceton gelöst, hierauf werden Polyäthylenglykol und Cetylpolyäthylenglykoläther zugesetzt. Die so erhaltene Lösung wird auf Kaolin aufgesprüht und anschliessend im Vakuum verdampft.

Spritzpulver

Zur Herstellung eines a) 50%igen, b) 25%igen und c) 10%igen Spritzpulvers werden folgende Bestandteile verwendet:

a) 50 Teile Wirkstoff gemäss Beispiel 1
5 Teile Natriumdibutylnaphthylsulfonat,
3 Teile Naphthalinsulfonsäuren-Phenonolsulfonsäuren-Formaldehyd-Kondensat 3:2:1,
20 Teile Kaolin,
22 Teile Champagne-Kreide;

b) 25 Teile des obigen Wirkstoffes,
5 Teile Oleylmethyltaurid-Natrium-Salz,
2,5 Teile Naphthalinsulfonsäuren-Formaldehyd-Kondensat,
0,5 Teile Carboxymethylcellulose,
5 Teile neutrales Kalium-Aluminium-Silikat,
62 Teile Kaolin;

c) 10 Teile des obigen Wirkstoffes,
3 Teile Gemisch der Natriumsalze von gesättigten Fettalkoholen,
5 Teile Naphthalinsulfonsäuren-Formaldehyd-Kondensat,
82 Teile Kaolin.

Der angegebene Wirkstoff wird auf die entsprechenden Trägerstoffe (Kaolin und Kreide) aufgezogen und anschliessend vermischt und vermahlen. Man erhält Spritzpulver von vorzüglicher Benetzbarkeit und Schwebefähigkeit. Aus solchen Spritzpulvern können durch Verdünnen mit Wasser Suspensionen jeder gewünschten Wirkstoffkonzentration erhalten werden. Derartige Suspensionen werden zur Bekämpfung von Ungräsern in Kulturpflanzungen verwendet.

Paste

Zur Herstellung einer 45%igen Paste werden folgende Stoffe verwendet:

45 Teile Wirkstoff
5 Teile Natriumaluminiumsilikat,
14 Teile Cetylpolyäthylenglykoläther mit 8 Mol Aethylenoxid,
1 Teil Oleylpolyäthylenglykoläther mit 5 Mol Aethylenoxid,
2 Teile Spindelöl,
23 Teile Wasser,
10 Teile Polyäthylenglykol.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Emulsionskonzentrat

Zur Herstellung eines 25%igen Emulsionskonzentrates werden

25 Teile Wirkstoff gemäss Beispiel 2
10 Teile Mischung von Nonylphenolpolyoxyäthylen und Calciumdodecylbenzol-sulfonat,
10 Teile Cyclohexanon,
55 Teile Xylol

miteinander vermischt. Dieses Konzentrat kann mit Wasser zu Emulsionen auf geeignete Konzentrationen verdünnt werden.

Anstatt des jeweiligen in den vorhergehenden Formulierungsbeispielen angegebenen Wirkstoffs kann auch eine andere von der Formel I umfassten Verbindungen verwendet werden.

Erfindungsgemässe Mittel, die als aktive Komponente mindestens einen Wirkstoff der Formel I enthalten, eignen sich besonders zur selektiven Bekämpfung monocotyler Ungräser in pre- und postemergenter Anwendung in dicotylen Kulturpflanzenbeständen, wie z.B. Soja, Baumwolle, Zuckerrübe, Leguminosen, Klee, Luzerner, Melone, Gurke, Tabak usw.

Zum Nachweis der Brauchbarkeit als Herbizide (pre- und postemergent) dienen folgende Testmethoden:

Pre-emergente Herbizid-Wirkung (Keimhemmung)

Im Gewächshaus wird unmittelbar nach der Einsaat der Versuchspflanzen in Saatschalen die Erdoberfläche mit einer wässerigen Dispersion der Wirkstoffe, erhalten aus einem 25%igen Spritzpulver oder Emulsionskonzentrat behandelt. Es wurden vier verschiedene Konzentrationsreihen angewendet, entsprechend 4, 2, 1 und 0,5 kg Wirksubstanz pro Hektar. Die Saatschalen werden im Gewächshaus bei 22-25°C und 50-70% rel. Luftfeuchtigkeit gehalten und der Versuch nach 3 Wochen ausgewertet und die Resultate nach folgender Notenskala bonitiert:

1 = Pflanzen nicht gekeimt oder total abgestorben
2-3 = sehr starke Wirkung
4-6 = mittlere Wirkung
7-8 = geringe Wirkung
9 = keine Wirkung (wie unbehandelte Kontrolle)

als Versuchspflanzen dienen:

| | |
|---|---|
| hordeum (Gerste) | setaria italica |
| triticum (Weizen) | echinochloa crus galli |
| zea (Mais) | beta vulgaris |
| sorghum hybr. (Hirse) | sida spinosa |
| oryza (Reis) | sesbania exaltata |
| glycine (Soja) | amaranthus retroflexus |
| gossypium (Baumwolle) | sinapis alba |

| | |
|---|---|
| avena fatua | ipomoea purpurea |
| lolium perenne | galium aparine |
| alopecurus myosuroides | pastinaca sativa |
| bromus tectorum | rumex sp. |
| cyperus exculentus | chrysanthemum leucum. |
| rottboellia exaltata | abutilon sp. |
| digitaria sanguinalis | solanum nigrum |

Post-emergente Herbizid-Wirkung (Kontaktherbizid)

Eine grössere Anzahl (mindestens 7) Unkräuter und Kulturpflanzen, sowohl monocotyle wie dicotyle, wurden nach dem Auflaufen (im 4- bis 6-Blattstadium) mit einer wässrigen Wirkstoffdispersion in Dosierungen von 0,25; 0,5; 1, 2 und 4 kg Wirksubstanz pro Hektar auf die Pflanzen gespritzt und diese bei 24°-26°C und 45-60% rel. Luftfeuchtigkeit gehalten. 15 Tage nach Behandlung wird der Versuch ausgewertet und das Ergebnis wie im preemergent-Versuch nach derselben Notenskala bonitiert.

Patentansprüche

1. Neue herbizid wirksame substituierte Diphenyläther-Derivate von der Formel I

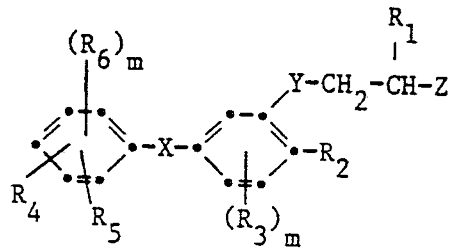

(I)

in welcher $R_1$ einen $C_1$-$C_4$ Alkylrest, $R_2$ Wasserstoff, Halogen oder die Cyanogruppe, $R_3$ Wasserstoff, Halogen, $C_1$-$C_4$ Alkyl oder die Nitrogruppe, $R_4$ und $R_5$ unabhängig voneinander je Wasserstoff, $-CF_3$, -CN oder $-NO_2$. $R_6$ Wasserstoff oder Halogen, m jeweils eine Zahl von 0 bis 3, X und Y unabhängig voneinander ein Sauerstoff- oder Schwefelatom und Z Halogen oder eine Gruppe -OH, -SH, $-OR_7$, $\underset{(O)_n}{\underset{\parallel}{-S}}-R_7$ oder

$$-N\begin{matrix} R_8 \\ R_9 \end{matrix}$$

bedeuten, in welch letzteren Gruppen die Reste folgende Bedeutung haben: $R_7$ = Alkyl, Cycloalkyl, Alkenyl, Cycloalkenyl, Alkinyl, Phenyl oder Benzyl, welche Reste noch durch Halogen, $CF_3$, $NO_2$, CN, Alkyl, Alkoxy, Alkylthio, -OH, $-NH_2$, Alkylamino, Dialkylamino und/oder Alkoxycarbonyl substituiert sein können, $R_7$ ferner eine -CO-NH-Alkyl-, -CO-NH-Aryl-, CO-N(Dialkyl), -CO-Aryl- oder -CO-Alkyl-Gruppe, wobei die Alkyl- und Arylreste noch durch Halogen etc. substituiert sein können, $R_7$ schliesslich auch ein Rest

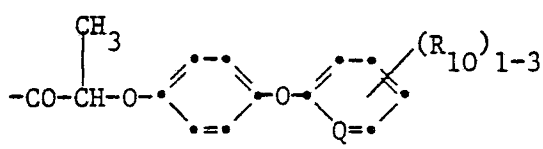

in welchem der oder die Substituenten $R_{10}$ unabhängig voneinander Halogen, $NO_2$, CN, $CF_3$ oder Alkyl darstellen und Q durch =CH- oder =N-

verkörpert ist, n die Zahl 0, 1 oder 2, $R_8$ und $R_9$ unabhängig voneinander je Wasserstoff, $C_1$-$C_4$ Alkyl, das noch durch Halogen, CN, OH oder $C_1$-$C_4$ Alkoxy substituiert sein kann, oder $R_8$ und $R_9$ bilden zusammen mit dem benachbarten Stickstoffatom einen 5- bis 6-gliedrigen heterocyclischen Ring, der noch ein Sauerstoff-, Schwefel- oder weiteres Stickstoffatom als zweites Heteroatom enthalten und durch $C_1$-$C_4$ Alkyl oder durch Phenyl substituiert sein kann.

2. Diphenylätherderivate gemäss Patentanspruch 1, von der Formel

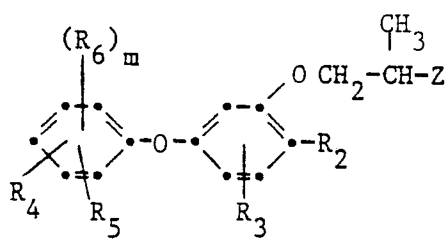

worin $R_2$ Wasserstoff, Halogen oder die Cyanogruppe, $R_3$ Wasserstoff oder Halogen bedeuten und die übrigen Radikale dieselbe Bedeutung wie im Patentanspruch 1 haben.

3. Diphenylätherderivate gemäss Patentanspruch 2, von der Formel

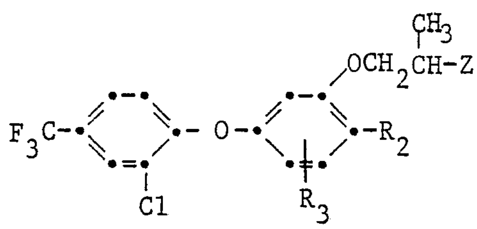

worin $R_3$ Wasserstoff oder Halogen ist und $R_2$ und Z wie unter Anspruch 1 definiert sind.

4. Diphenylätherderivate der Formel

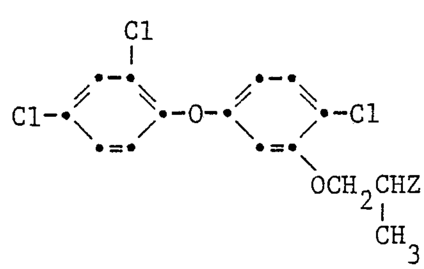

worin Z die unter Patentanspruch 1 gegebenen Bedeutungen besitzt.

5. Diphenylätherderivate der Formel

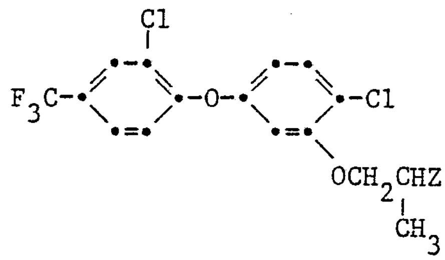

worin Z wie im Patentanspruch 1 definiert ist.

6. Diphenylätherderivate der Formel

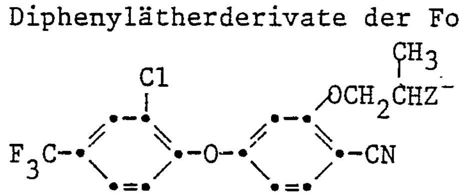

worin Z wie im Patentanspruch 1 definiert ist.

7. Die Verbindung der Formel

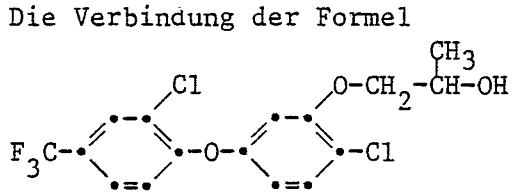

8. Herbizides Mittel, dadurch gekennzeichnet, dass es als wirksame Komponente ein Diphenylätherderivat der Formel I des Patentanspruchs 1 enthält.

9. Die Verwendung der Verbindungen bzw. Mittel gemäss Ansprüchen 1 bis 8 zur Bekämpfung von monocotylen Unkräutern.

10. Verwendung gemäss Patentanspruch 9 zur selektiven Bekämpfung von Ungräsern in Kulturpflanzenbeständen.

Europäisches Patentamt

# EUROPÄISCHER RECHERCHENBERICHT

0018324

EP 80 81 0118

| Kategorie | EINSCHLÄGIGE DOKUMENTE<br>Kennzeichnung des Dokuments mit Angabe, soweit erforderlich, der maßgeblichen Teile | betrifft Anspruch |
|---|---|---|
| A | FR - A - 2 245 613 (MITSUI TOATSU CHEMICALS)<br>* Anspruch 1 *<br>-- | 1 |
| A | EP - A - 0 003 295 (CIBA-GEIGY)<br>* Anspruch 1 *<br>-- | 1 |
| A | DE - A - 2 809 541 (CIBA-GEIGY)<br>* Anspruch 1 *<br>-- | 1 |
| A | US - A - 4 093 446 (H.O. BAYER et al.)<br>* Ansprüche 1,2 *<br>---- | 1 |

KLASSIFIKATION DER ANMELDUNG (Int.Cl.)

C 07 C 43/257
41/16
125/065
121/75
149/00
147/00
69/96
69/712
C 07 D 213/64
A 01 N 31/16
33/04
47/20

RECHERCHIERTE SACHGEBIETE (Int. Cl.)

C 07 C 43/295
43/29
41/16

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 25-07-1980 | SAGATYS |